# EUROPEAN PATENT SPECIFICATION

(11) **EP 2 422 746 B1**
(45) Date of publication and mention of the grant of the patent: **19.07.2017**
(21) Application number: 10251496.5
(22) Date of filing: 26.08.2010
(51) Int. Cl.: A61F 2/16

(54) **Intraocular implant**
Intraokulares Implantat
Implant intra-oculaire

(43) Date of publication of application: 29.02.2012
(60) Divisional application: 16197820.0
(73) Proprietor: Stevens, Julian Douglas, London N1 7RH (GB)
(72) Inventor: Stevens, Julian Douglas, London N1 7RH (GB)
(74) Representative: Miller Sturt Kenyon

(56) References cited:
- WO-A1-2007/082342
- US-A- 4 124 905
- US-A- 4 342 123
- US-A- 5 171 320
- US-A- 5 476 512
- US-A- 5 697 973
- US-A1- 2006 047 339

## Description

### FIELD OF THE INVENTION

The invention relates to an intraocular implant, and in particular to an intraocular implant for use in cataract operations or refractive crystalline lens extraction operations generally.

### BACKGROUND OF THE INVENTION

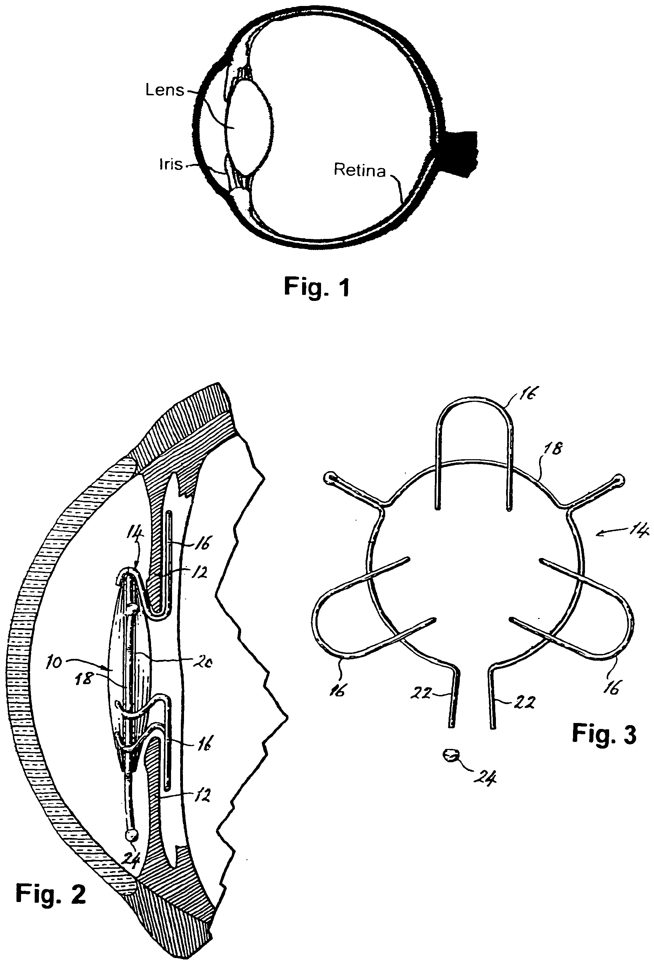

The cataract condition is a well known eye ailment, which these days is easily treatable through surgery. The condition involves an opacification of the lens (see Fig. 1), which is situated just behind the iris and serves to focus the incoming image onto the retina at the back of the eye.

The surgical procedure involves the removal of the opaque lens and its substitution by an artificial lens having the required focusing effect.

One way of achieving this is described in U.S. patent no. 3,925,825. Figs. 2 and 3 correspond to Figs. 1 and 2 of this patent, and show the placing of a lens 10 anterior to the iris 12. The lens is held in place against the iris by a haptic section 14. The haptic section 14 consists of a series of bent wire loops 16, which are attached to a circular wire frame 18. The lens 10 has a flat circumferential face 20 and the lens is held within the frame 18 by inserting the lens inside the frame 18 and closing the legs 22 of the frame so that the frame 18 closes tightly against the lens face 20. Once the legs 22 have been brought together, their ends are kept together via a terminal part 24.

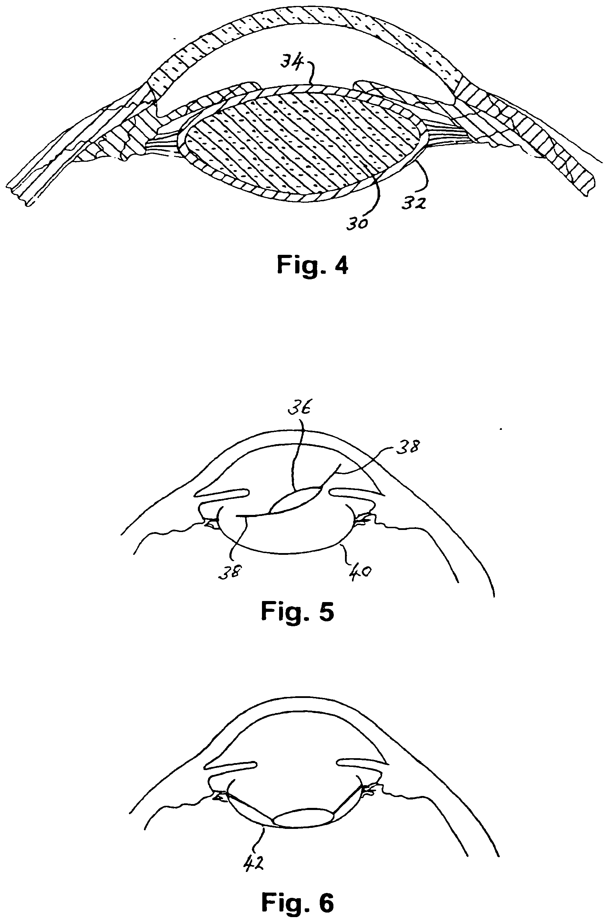

One way of extracting the opaque natural lens - a process known as extra-capsular extraction - will now be described. Reference is first made to US 2003/0130732, Fig. 1 of which corresponds to Fig. 4 of the present application. The lens 30 is accommodated within a so-called capsule, which is shown as item 32 in Fig. 4. To remove the opaque lens, an opening (a so-called "capsulotomy") is made in the anterior part 34 of the capsule 32 manually by a surgeon or by a pulsed laser and the lens 30 is removed through the opening.

To facilitate the removal of the opaque lens, the lens is first emulsified by the phacoemulsification method or by a pulsed laser. Phacoemulsification involves making a small incision in the cornea and introducing a very thin needle through the incision, which is then brought into contact with the lens through the capsulotomy. The needle is caused to vibrate at an ultrasonic frequency by the use of a magnetostrictive driver. The ultrasonic vibrations of the needle soften the lens and emulsify it. The emulsified parts can then be aspirated out of the capsule through the incision. Finally, the incision is widened sufficiently to introduce the substitute artificial lens into the capsule.

A pulsed laser can be used to create an opening in the capsule, by photoablating capsular tissue along a boundary having a predetermined profile, e.g., circular, oval or elliptical.

Like the lens of Fig. 2, the lens in the extra-capsular method is conventionally held within the capsule by the use of haptics. One example of this is shown in US 5,376,115. Figs. 5 and 6 are an extract from this patent, in which the artificial lens 36 with its haptics 38 is introduced through the iris and into the capsule 40 via the capsulotomy (Fig. 5) and finally brought to bear against the inside posterior surface of the capsule (Fig. 6). The haptics 38 are used to center the lens and secure it in place inside the capsule.

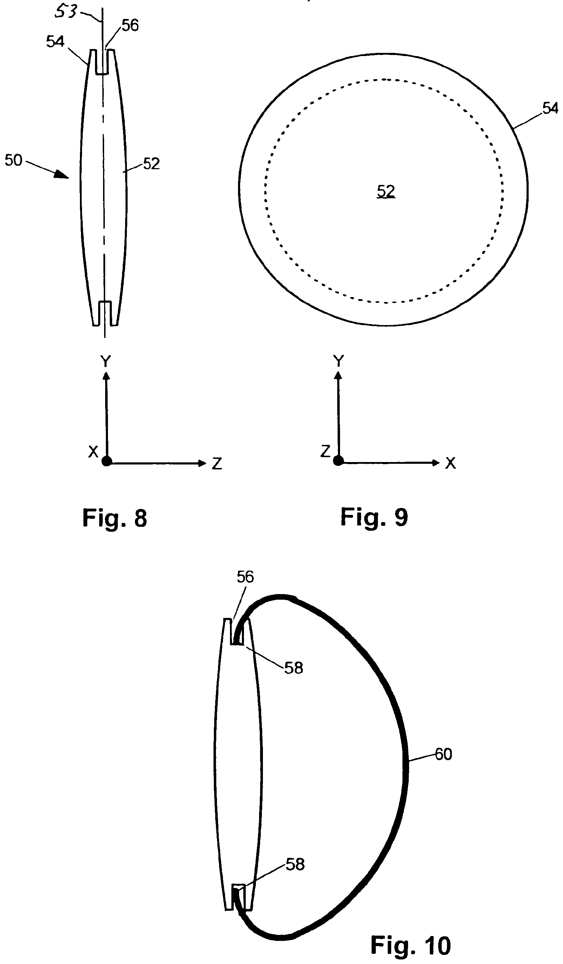

One problem associated with these known capsular insert techniques is that it is difficult to centre the lens accurately in the X-Y plane of the eye. The X-Y-Z co-ordinates are shown in Figs. 8 and 9, where the radius of the lens lies in the X-Y plane and the Z-direction is parallel to the optical axis of the eye. A second problem is that it can be difficult to properly define the position of the lens in the Z-direction. This can lead to difficulties in accurately defining the optical power required for the artificial lens. A third problem, which one embodiment of the present invention can solve, is that, some time after the cataract operation has been carried out, the posterior wall of the capsule (the "posterior capsule") can become opaque like the original lens, requiring a further surgical intervention.

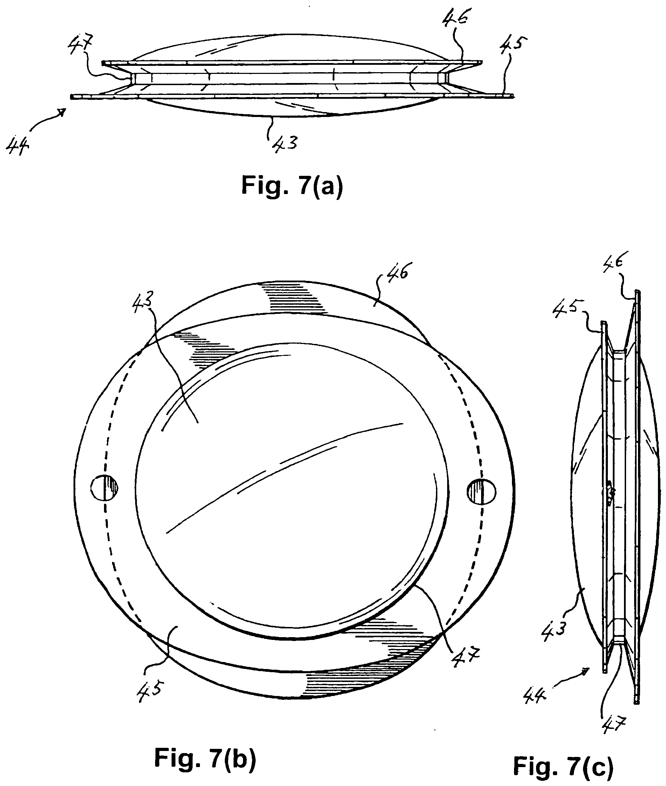

United States Patent no. 6,027,531 describes a so-called "bag-in-lens" technique, in which the optical implant takes the form shown in Figs. 7(a)-7(c). This implant comprises a lens optic 43 and a haptic portion 44 comprising an anterior lip 45 and a posterior lip 46. The haptic portion 44 is configured to form a groove 47, into which will be fitted the lips of a pair of capsulotomies formed respectively in the anterior and posterior walls of the capsule.

US 4,124,905 describes an intraocular implant for use with the iris. The implant comprises a main portion, which is an optical zone, and a peripheral portion in the form of two oppositely disposed tabs forming a groove for engagement with the iris. The groove lies substantially parallel to a plane of the main portion. Also included is a pin, which is pivotally supported in the implant and whose free end can be received by an opening formed in the peripheral portion. In use, the implant is inserted into the iris and the free end of the pin moved into the opening, while puncturing the iris seated in the groove.

In US 2006/0047339 an intraocular implant comprises a groove for engagement with an anterior or posterior capsulotomy. The groove is parallel with the plane of the implant. Eyelets are provided, so that the implant can be sutured to the iris or other eye tissue, and cleats, which are integral to the peripheral part of the implant, enable a replaceable lens to be secured to the implant. Thus the implant has a separate lens and frame. An alternative arrangement employs a series of spaced-apart tabs instead of the groove. A similar cleat system is used here also for the securing of a detachable lens to the implant.

US 5,697,973 discloses an intraocular silicone lens in which an annular ring includes an upper flange and a lower flange connected by an inner wall. Together, these form a channel around the lens, which is said to allow the lens to be fixated to the periphery of the iris or an opening in the capsular bag.

### SUMMARY OF THE INVENTION

The present invention provides an intraocular implant as defined, in a first aspect of the present invention, in claim 1.

The second groove may be of smaller mean diameter than the first groove.

The groove may extend continuously around the peripheral portion of the implant. Alternatively, it may be provided at discrete locations around the peripheral portion of the implant, in which case the discrete locations are preferably substantially equidistant from each other.

The groove may be provided as a gap between a first projection from the main portion of the implant and a second projection from the main portion of the implant. Either the first projection and/or the second projection may be provided as a plate or as a loop, or the first projection is provided as a plate and the second projection is provided as a loop. The plate or loop is preferably rounded at its end.

The implant may further comprise at the peripheral portion thereof at least one lug for engagement with corresponding voids formed in the capsule. Two, three or four lugs may be provided spaced around the peripheral portion for engagement with respective voids formed in the capsule. The lugs are preferably substantially equidistantly placed around the peripheral portion of the implant.

The implant may be a lens.

### BRIEF DESCRIPTION OF THE DRAWINGS

Embodiments of the present invention will now be described, by way of example only, with the aid of the drawings, of which:
Fig. 1 is a sectional view of the human eye;
Fig. 2 is a part-side and part-sectional view of a human eye with an artificial lens substituted for the original lens in accordance with a known method;
Fig. 3 shows a haptic arrangement used in the known method of Fig. 2;
Fig. 4 is a sectional view of a human eye showing the natural lens in its capsule;
Figs. 5 and 6 are sectional views showing two stages in a known lens replacement method employing extra-capsular extraction;
Figs. 7(a)-7(c) are orthogonal views of a known "bag-in-lens" implant;
Figs. 8 and 9 are side and front views, respectively, of a first example of an intraocular insert;
Fig. 10 is the side view according to Fig. 8 with the lens engaged with the capsule;
Figs. 11(a) and 11(b) are a side view of a first embodiment according to the invention;
Figs. 12(a), 12(b) and 12(c) are side and front views of a variant of the first embodiment according to the invention;
Fig. 13 is a side view of a yet further variant of the first embodiment according to the invention;
Figs. 14 and 15 are front and plan views, respectively, of a second example of an intraocular insert;
Figs. 16(a) and 16(b) are different realizations of the projections shown in Figs. 14 and 15;
Figs. 17 and 18 are front and plan views, respectively, of a variant of the second example;
Fig. 19 shows two different examples of a third example of an intraocular implant;
Fig. 20 is a plan view of an intraocular implant illustrating the principle of a fourth example;
Figs. 21 and 22 are plan and front views, respectively, of one realization of the fourth example, and
Figs. 23 and 24 are plan and front views, respectively, of a second realization of the fourth example.

### DETAILED DESCRIPTION OF THE EMBODIMENTS

A first example of an intraocular implant not in accordance with the present invention is illustrated in Figs. 8 and 9, in which Fig. 8 is a side view and Fig. 9 is a front view of the implant.

The implant in this example is an artificial lens implant 50 for treating, e.g., cataracts, and comprises a lens body portion 52, which is the lens proper (the "optic"), and a peripheral portion 54. The peripheral portion 54 has formed in an axially approximately central part thereof (i.e. referred to the Z-axis) a groove 56, which extends in a radial direction from the periphery of the implant toward its optical axis. Thus, the groove 56 lies parallel to, and preferably in, the axially central plane 53 of a lamina constituted by the optic 52. The term "lamina" refers to a plate-like member having a surface area much greater than its thickness, especially at its outer edge. During the surgical procedure, the lens 50 is offered up to the anterior capsulotomy, which has been formed by laser in the capsule 60 (see Fig. 10), as mentioned above in connection with the known implantation method, and the lip 58 of the capsulotomy is fitted into the groove 56, thereby holding the implant in place. Hence, this method dispenses with the need for haptics, instead relying on the lens itself for securing to the capsule. In addition, the correct positioning of the lens vis-à-vis the optical axis of the eye can be ensured by accurately locating the capsulotomy in the X-Y plane (see Figs. 8 and 9). Thus, some eccentricity can be introduced into the radial positioning of the lens by this appropriate locating of the capsulotomy in the capsule.

It is desirable not only that the capsulotomy be accurately located in the capsule, but also that the size of the capsulotomy be accurately defined, so that it can fit the groove in the implant. This is possible at this present time, due to the existence of pulsed ultrafast femtosecond laser technology, by means of which extremely accurate cutting operations can be achieved in a reliable and repeatable manner.

Where desired, one or more fenestrations can be made in the capsule, in order to provide for the passage of a lens or device into the capsular bag or to allow the transfer of aqueous fluid.

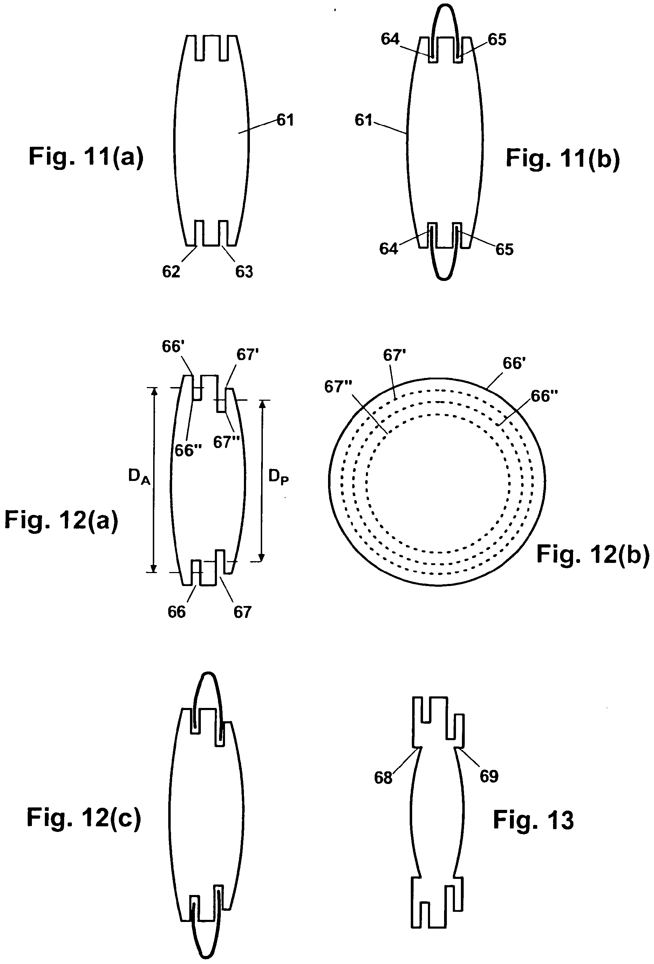

Although this example has been described in connection with an anterior capsulotomy, in practice the implant may be made to engage with a posterior capsulotomy instead, or even with both types of capsulotomy at the same time. A realization of this latter arrangement is illustrated in Figs. 11a and 11b, in which an implant comprises the optic portion 61 and a peripheral portion with a pair of annular grooves 62 and 63. These grooves are spaced apart from each other in the Z-direction (see Fig. 8) and are designed to take respective lips 64, 65 of an anterior and posterior capsulotomy (see Fig. 11b). A variant version of this arrangement is shown in the side and front views of Figs. 12a and 12b, respectively. In this version the posterior capsulotomy is smallerthan the anterior capsulotomy. To accommodate this, the anterior groove 66 has a larger mean periphery than the posterior groove 67. This is best explained with reference to Fig. 12b, in which the larger groove has an outer periphery 66' and an inner periphery 66", while the smaller groove has an outer periphery 67' and an inner periphery 67". The mean periphery of the groove 66 is clearly larger than that of the groove 67. For a circular profile, as illustrated, this means that the mean anterior diameter D_{A} of the groove 66 is larger than the mean posterior diameter D_{P} of the groove 67.

This version as fitted to the bag is shown in Fig. 12c.

The Fig. 12 arrangement can be beneficial to a patient, since it is often desirable to limit the extent of the posterior capsulotomy, as this reduces risk of the lens implant being accidentally displaced through the posterior capsulotomy into the vitreous cavity.

One potential drawback of the arrangements illustrated in Figs. 11 and 12 is that the provision of two grooves in the same lens can increase the thickness of the optic. To avoid this, the configuration of Fig. 13 may be employed. In this configuration the peripheral portion of the implant has either or both of an anterior overhang 68 and a posterior overhang 69, which allows a reduced thickness of the optic.

This first embodiment also envisages the use of a complex-lens arrangement involving the use of more than one optic. In particular, two optics may be used, which are connected together to form a single unit. This apparatus may be fixated to the anterior capsulotomy or to a posterior capsulotomy, or to both. In the latter case, the two capsulotomies engage with two different spaced-apart grooves.

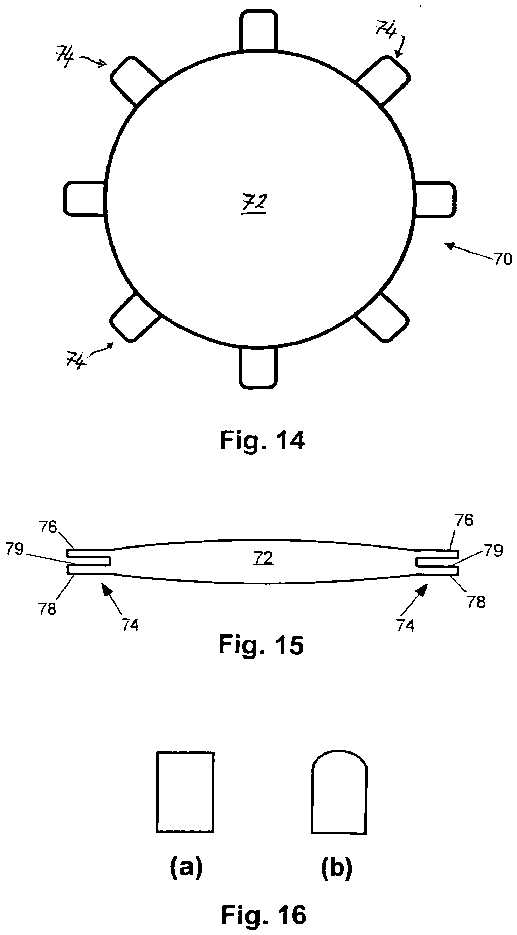

A second example is illustrated in Figs. 14 and 15. Here a lens 70 has a optic portion 72 and a series of projection portions 74 projecting from the circumference of the lens portion. Referring to the plan view of Fig. 15, each projection portion comprises a first projection 76 and a second projection 78, which are disposed at opposite sides of the lens, and a gap 79 between the first and second projections. The gap 79 corresponds to the groove 54 in Fig. 8. The projections 76 and 78 may be both rectangular or rounded in shape, or a combination of both (see Figs. 16(a) and 16(b)). Hence the projections 76 may be rectangular, while the projections 78 are rounded, or vice-versa, or any combination of the two around the periphery of the implant, or the projections may be all of one type. In practice, the rounded configuration is preferred for all of the projections, since this makes it easier to fit the capsulotomy to the implant and also reduces the risk of trauma to the capsule, that a sharp edge of the rectangular profile might cause. The projections are either integral to the optic (i.e. moulded), or they may be attached to the body of the lens by some suitable means (e.g. mechanical slot and groove) and can be of the same material as the lens optic or of a different bio-material. The projections are either in the form of thin loops or plates. Although eight projection pairs have been shown, the invention is not limited to this number. More or less than eight may be used. The criterion for selecting the appropriate number is the reliability and stability of the attachment of the lens to the capsule.

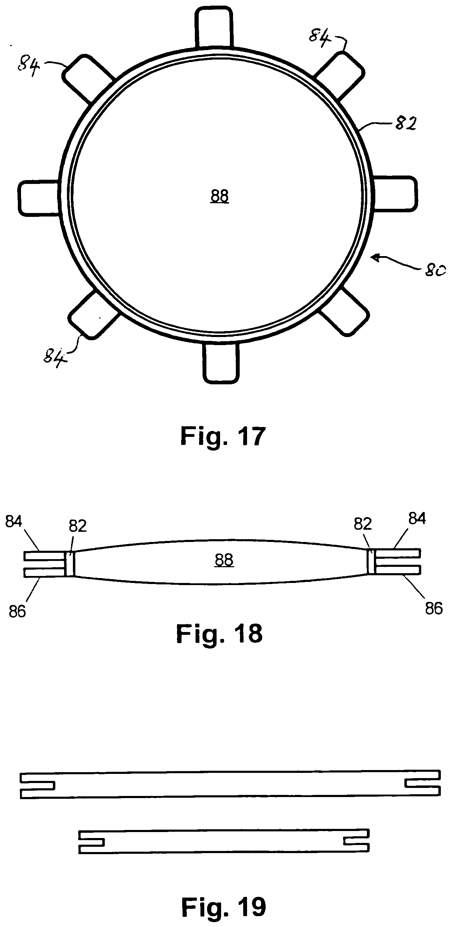

In a variant of the example just described, the projections are not integral with the optic, or attached directly to the optic, but instead form part of a separate projection unit, which is fitted to the optic. This is shown in Figs. 17 and 18, in which the projection unit 80 comprises an annular frame 82, which is tighly fitted to the optic 88, and attached to which are pairs of projections 84, 86.

In a third example, the intraocular implant of the first example is not a lens, but a plug or bung. Such an implant is not required to have any optical power, but serves merely to close a fenestration or other aperture in the capsule. An example of this is shown in Fig. 19. Indeed, Fig. 19 shows two examples of different diameter, depending on the size of the fenestration to be closed. It can be seen that this implant is flat and therefore has no optical power. Depending on the position of the fenestration being closed, the plug may also be opaque.

Fenestrations that might be closed off using the plug are, for example, fenestrations introduced into the capsule in order to facilitate the entry of an instrument for evacuating the lens material. In this respect, femtosecond lasers are often used to cut the lens into very small cubes or slices, so that they can be evacuated through a small-bore instrument or cannula. The cannula can be introduced into the capsule through such a fenestration. In addition, the plug can be used to provide tectonic support, in order to keep compartments in the eye physically separate. It is also useful as a means of preventing silicone oil from moving forward into the anterior segment of the eye.

A fourth example will now be described.

The fourth example recognizes that there is often a need to rotationally align a lens implant, where there is an asymmetrical optic (e.g. where there is variable optical power in the lens or where there is a toric optic). This example utilizes forward or backward facing lugs, which are placed in voids (e.g. fenestrations or very small capsulotomy holes) in the capsule. These voids are located radially outside the capsulotomy and can be formed by photoablation via a laser.

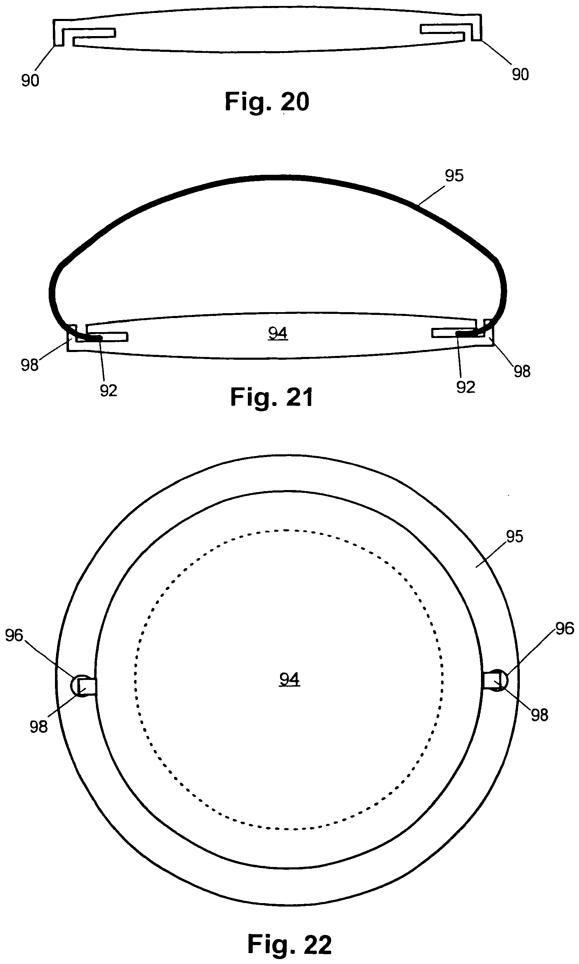

The principle of this example is illustrated in Fig. 20. In Fig. 20 a lens implant such as shown in Fig. 8 or Fig. 15 is provided with one or more lugs 90 situated radially outwardly of the outer ends of the groove 56 or projections 76, 78.

Figs. 21 and 22 illustrate one specific realization of this example, based on the first example. As in Fig. 10, the lip 92 of the capsulotomy in this example is engaged in the groove of the lens 94. Holes 96 have been formed in the capsule 95 and a pair of oppositely disposed lugs 98, which are integral with the lens, are made to engage with these holes, thereby securing the lens in a given rotational position relative to the capsule. In this arrangement the implant is oriented so that the lugs face toward the back of the eye. Instead, the lens may be reversed, so that the lugs face toward the front of the eye and therefore engage with the residual anterior capsule from an inside surface thereof instead of from the outside surface thereof, as in Fig. 21.

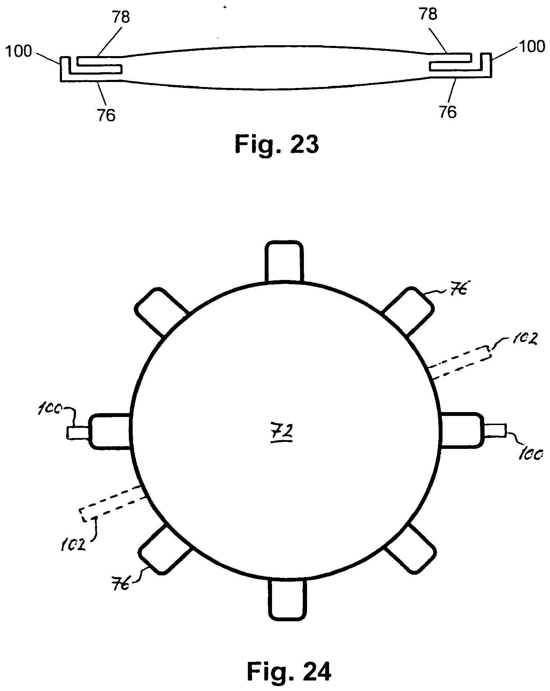

A variant of this arrangement is shown in Figs. 23 and 24, and is based on the second example. Here a pair of oppositely placed lugs 100 is provided as integral extensions to two of the first projections 76 or second projections 78. These engage with holes or voids in the capsule similar to those shown in Fig. 22. In a further variant of this variant arrangement, the lugs are instead each provided between two adjacent projections 76 or 78, as shown by the dotted-line projections 102 in Fig. 24. These projections 102 are therefore dedicated solely to carrying the lugs, whereas in the first arrangement the projections 76 (or 78) act both as protrusions for engagement with the lip of the capsulotomy and as carriers of the lugs for engagement with the capsule.

Although the lugs shown in Figs. 20, 21 and 23 are of a length such as to end up approximately flush with the radially outer part of the projecting portion - e.g. flush with the projections 78 in Fig. 23, and similarly in Fig. 21 - in practice the lugs will have a length such as to enable them to be readily fitted into the voids in the capsule wall, with at the same time the capsule lip being fitted into the groove(s). As shown in Fig. 21, the lugs preferably pass right through through-holes made in the capsule wall, and will therefore be of a length, which enables them to do that, while not being so long that they make it difficult to fit the capsule lip into the groove(s).

As regards the end-profile of the lugs (that is, looking down onto the end of the lugs), this is preferably circular, though other profiles may also be suitable, provided they are able to engage with the voids made in the capsule.

Instead of two lugs per lens, only one may be employed, or more than two - e.g. three or four. They will generally be equally distributed around the periphery of the lens.

The advantages of the present invention are as follows:
◆ accurate positioning of the lens in the X-Y plane over the pupil centre or any desired centre-point (e.g. the line of sight);
◆ potentially more accurate placement in the Z-direction, due to the siting of the lens in the anterior or posterior part of the capsule;
◆ if the lens is supported by the posterior part of the capsule, then the opacification, to which the posterior capsule is often subject post-op, cannot occur;
◆ the insert can take the form of a plug instead of a lens, which can be used to seal a capsular opening used during surgery;
◆ such a plug could comprise a valve arrangement to allow the passage of fluid, which could regulate the volume of the capsule;
◆ because there is predictable positioning of the lens in the Z-direction, then a more reliable biometry formula can be written;
◆ since the insert is secured by the rim of the capsulotomy, its rotational position can be defined by the forming of marker voids or fenestrations in the capsule and of lugs as extensions to the lens, which engage with the fenestrations;
◆ the provision of two grooves axially spaced apart in the implant allows anterior and posterior capsulotomies of different sizes to be engaged with the implant, since each capsulotomy is allocated to its own groove.

Although the implant has been described for use with the human eye, it could equally well be used with the animal eye.

In addition, although implants of circular profile have been shown in connection with the various embodiments, the profile used can be any desired profile thought to be appropriate to the situation in question. Thus, oval or elliptical profiles are possible, for instance.

As regards the materials used for the implant any of the materials used for intraocular lens implants, such as acrylic silicone or hydrogel, are suitable.

The implant of the present invention has been described in connection with its use in cataract operations. However, it may also be used for treating myopia, hyperopia, astigmatism or presbyopia (refractive lens exchange surgery).

The foregoing description has been given by way of example only and it will be appreciated by a person skilled in the art that modifications can be made without departing from the scope of the present invention.

## Claims

1. An intraocular implant for placement in the eye, the implant comprising a main portion (61), which is a lamina, and a peripheral portion disposed at the periphery of the main portion, the peripheral portion comprising a groove for engagement with the lip of a single capsulotomy only formed in the lens capsule of the eye, the groove lying substantially parallel to the plane of the lamina;
**characterized in that**:
the groove is constituted by first and second grooves (66, 67) lying parallel to the plane of the lamina and spaced apart from each other in a direction orthogonal to said plane, the first groove (66) being for engagement with the lip of an anterior capsulotomy and the second groove (67) being for engagement with the lip of a posterior capsulotomy.

2. An intraocular implant as claimed in claim 1, wherein
the implant comprises a multi-lamina unit comprising first and second laminae spaced apart from each other in a direction orthogonal to the planes of the laminae, and
the first lamina has the first groove and the second lamina has the second said groove.

3. An intraocular implant as claimed in claim 1 or claim 2, wherein the second groove is of smaller mean circumference than the first groove.

4. An intraocular implant as claimed in any one of the preceding claims, wherein the grooves extend continuously around the peripheral portion of the implant.

5. An intraocular implant as claimed in any one of the preceding claims, wherein the grooves are provided at discrete locations around the peripheral portion of the implant.

6. An intraocular implant as claimed in claim 5, wherein the discrete locations are equidistant from each other.

7. An intraocular implant as claimed in claim 5 or claim 6, wherein the grooves are provided as a gap between a first projection from the main portion of the implant and a second projection from the main portion of the implant.

8. An intraocular implant as claimed in claim 7, wherein either the first projection and/or the second projection is provided as a plate.

9. An intraocular implant as claimed in claim 7, wherein either the first projection and/or the second projection is provided as a loop.

10. An intraocular implant as claimed in claim 7, wherein the first projection is provided as a plate and the second projection is provided as a loop.

11. An intraocular implant as claimed in any one of claims 8 to 10, wherein the plate or loop is rounded at its end.

12. An intraocular implant as claimed in any one of the preceding claims, wherein the implant further comprises at the peripheral portion thereof at least one lug for engagement with corresponding voids formed in the capsule.

13. An intraocular implant as claimed in claim 12, comprising two, three or four lugs spaced around the peripheral portion for engagement with respective voids formed in the capsule.

14. An intraocular implant as claimed in claim 13, wherein the lugs are equidistantly placed around the peripheral portion of the implant.

15. An intraocular implant as claimed in any one of the preceding claims, wherein the implant is a lens.

## Patentansprüche

1. Intraokulares Implantat zur Auflagerung im Auge, wobei das Implantat einen Hauptabschnitt (61) umfasst, der eine Lamina ist, und einen Umfangsabschnitt, der am Umfang des Hauptabschnitts angeordnet ist, wobei der Umfangsabschnitt eine Nut zum Eingriff mit der Lippe aufweist Einer einzigen Capsulotomie, die nur in der Linsenkapsel des Auges gebildet ist, wobei die Rille im wesentlichen parallel zur Ebene der Lamina liegt;
**dadurch gekennzeichnet**:
wobei die Nut durch erste und zweite Nuten (66, 67) gebildet ist, die parallel zu der Ebene der Lamelle liegen und in einer Richtung orthogonal zu dieser Ebene voneinander beabstandet sind, wobei die erste Nut (66) zum Eingriff mit der Lippe eines Eine vordere Capsulotomie und die zweite Rille (67) zum Eingriff mit der Lippe einer hinteren Capsulotomie.

2. Intraokulares Implantat nach Anspruch 1, **dadurch gekennzeichnet**,
wobei das Implantat eine Multilamina-Einheit umfasst, die eine erste und eine zweite Schicht aufweist, die in einer Richtung orthogonal zu den Ebenen der Lamellen voneinander beabstandet sind, und
die erste Lamina hat die erste Nut und die zweite Lamina hat die zweite Nut.

3. Intraokulares Implantat nach Anspruch 1 oder 2, **dadurch gekennzeichnet, dass** die zweite Rille einen kleineren mittleren Umfang als die erste Rille aufweist.

4. Intraokulares Implantat nach einem der vorhergehenden Ansprüche, wobei sich die Rillen kontinuierlich um den Umfangsabschnitt des Implantats erstrecken.

5. Intraokulares Implantat nach einem der vorhergehenden Ansprüche, **dadurch gekennzeichnet, dass** die Rillen an diskreten Stellen um den Umfangsabschnitt des Implantats herum vorgesehen sind.

6. Intraokulares Implantat nach Anspruch 5, **dadurch gekennzeichnet, dass** die diskreten Stellen äquidistant zueinander sind.

7. Intraokulares Implantat nach Anspruch 5 oder 6, **dadurch gekennzeichnet, dass** die Rillen als Spalt zwischen einem ersten Vorsprung aus dem Hauptteil des Implantats und einem zweiten Vorsprung aus dem Hauptteil des Implantats vorgesehen sind.

8. Intraokulares Implantat nach Anspruch 7, **dadurch gekennzeichnet, dass** entweder der erste Vorsprung und/oder der zweite Vorsprung als Platte vorgesehen ist.

9. Intraokulares Implantat nach Anspruch 7, **dadurch gekennzeichnet, dass** entweder die erste Projektion und/oder die zweite Projektion als Schleife vorgesehen ist.

10. Intraokulares Implantat nach Anspruch 7, **dadurch gekennzeichnet, dass** der erste Vorsprung als Platte vorgesehen ist und der zweite Vorsprung als Schlaufe vorgesehen ist.

11. Intraokulares Implantat nach einem der Ansprüche 8 bis 10, **dadurch gekennzeichnet, dass** die Platte oder Schleife an ihrem Ende abgerundet ist.

12. Intraokulares Implantat nach einem der vorangehenden Ansprüche, wobei das Implantat ferner an seinem Umfangsabschnitt mindestens einen Ansatz zum Eingriff mit entsprechenden in der Kapsel gebildeten Hohlräumen aufweist.

13. Intraokulares Implantat nach Anspruch 12, das zwei, drei oder vier Laschen aufweist, die um den Umfangsabschnitt herum angeordnet sind, um mit entsprechenden Hohlräumen in der Kapsel in Eingriff zu kommen.

14. Intraokulares Implantat nach Anspruch 13, **dadurch gekennzeichnet, dass** die Laschen äquidistant um den Umfangsabschnitt des Implantats herum angeordnet sind.

15. Intraokulares Implantat nach einem der vorhergehenden Ansprüche, wobei das Implantat eine Linse ist.

## Revendications

1. Implant intraoculaire pour le placement dans l'oeil, l'implant comprenant une partie principale (61), qui est une lame, et une partie périphérique disposée à la périphérie de la partie principale, la partie périphérique comprenant une rainure pour l'engagement avec la lèvre D'une seule capsulotomie formée uniquement dans la capsule de l'objectif de l'oeil, la rainure étant sensiblement parallèle au plan de la lame;
**caractérisé en ce que**:
la rainure est constituée par des première et seconde rainures (66, 67) situées parallèlement au plan de la lame et espacées l'une de l'autre dans une direction orthogonale audit plan, la première rainure (66) étant en prise avec la lèvre d'un La capsulotomie antérieure et la seconde rainure (67) étant en contact avec la lèvre d'une capsulotomie postérieure.

2. Implant intraoculaire selon la revendication 1, dans lequel
l'implant comprend une unité à plusieurs lames comprenant des première et seconde lames espacées l'une de l'autre dans une direction orthogonale aux plans des lames et
la première lame a la première rainure et la deuxième lame a la seconde rainure.

3. Implant intraoculaire selon la revendication 1 ou la revendication 2, dans lequel la seconde rainure présente une circonférence moyenne plus petite que la première rainure.

4. Implant intraoculaire selon l'une quelconque des revendications précédentes, dans lequel les rainures s'étendent en continu autour de la partie périphérique de l'implant.

5. Implant intraoculaire selon l'une quelconque des revendications précédentes, dans lequel les rainures sont prévues à des emplacements discrets autour de la partie périphérique de l'implant.

6. Implant intraoculaire selon la revendication 5, dans lequel les emplacements discrets sont équidistants l'un de l'autre.

7. Implant intraoculaire selon la revendication 5 ou la revendication 6, dans lequel les rainures sont prévues en tant qu'intervalle entre une première projection de la partie principale de l'implant et une seconde projection de la partie principale de l'implant.

8. Implant intraoculaire selon la revendication 7, dans lequel la première projection et/ou la seconde projection sont prévues sous forme de plaque.

9. Implant intraoculaire selon la revendication 7, dans lequel la première projection et/ou la seconde projection sont prévues sous la forme d'une boucle.

10. Implant intraoculaire selon la revendication 7, dans lequel la première projection est prévue en tant que plaque et la seconde projection est prévue en boucle.

11. Implant intraoculaire selon l'une quelconque des revendications 8 à 10, dans lequel la plaque ou la boucle est arrondie à son extrémité.

12. Implant intraoculaire selon l'une quelconque des revendications précédentes, dans lequel l'implant comprend en outre sur sa partie périphérique au moins une patte pour l'engagement avec des vides correspondants formés dans la capsule.

13. Implant intraoculaire selon la revendication 12, comprenant deux, trois ou quatre pattes espacées autour de la partie périphérique pour l'engagement avec des vides respectifs formés dans la capsule.

14. Implant intraoculaire selon la revendication 13, dans lequel les pattes sont placées de manière équidistante autour de la partie périphérique de l'implant.

15. Implant intraoculaire selon l'une quelconque des revendications précédentes, dans lequel l'implant est une lentille.
